(19) Europäisches Patentamt
European Patent Office
Office européen des brevets

(11) **EP 3 842 789 B1**

(12) **EUROPEAN PATENT SPECIFICATION**

(45) Date of publication and mention
of the grant of the patent:
**16.10.2024 Bulletin 2024/42**

(21) Application number: **19851219.6**

(22) Date of filing: **20.08.2019**

(51) International Patent Classification (IPC):
**G01N 21/41** (2006.01)     **G01N 33/543** (2006.01)
**G01N 33/545** (2006.01)     **G01N 33/553** (2006.01)
**G01N 33/558** (2006.01)

(52) Cooperative Patent Classification (CPC):
**G01N 21/648; G01N 33/54373; G01N 33/54388;
G01N 33/585; G01N 33/587**

(86) International application number:
**PCT/JP2019/032510**

(87) International publication number:
**WO 2020/040159 (27.02.2020 Gazette 2020/09)**

(54) **IMMUNOCHROMATOGRAPHY IN WHICH CARRIER PARTICLES ARE USED TO AMPLIFY SURFACE PLASMON RESONANCE**

IMMUNOCHROMATOGRAPHIE, BEI DER TRÄGERPARTIKEL ZUR VERSTÄRKUNG DER OBERFLÄCHENPLASMONENRESONANZ VERWENDET WERDEN

IMMUNOCHROMATOGRAPHIE DANS LAQUELLE DES PARTICULES DE SUPPORT SONT UTILISÉES POUR AMPLIFIER LA RÉSONANCE PLASMONIQUE DE SURFACE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **21.08.2018 JP 2018154995**

(43) Date of publication of application:
**30.06.2021 Bulletin 2021/26**

(73) Proprietor: **Denka Company Limited
Tokyo 103-8338 (JP)**

(72) Inventor: **MATSUDA Yusuke
Gosen-shi, Niigata 959-1695 (JP)**

(74) Representative: **Clarenbach, Carl-Philipp et al
Gleiss Große Schrell und Partner mbB
Patentanwälte Rechtsanwälte
Leitzstraße 45
70469 Stuttgart (DE)**

(56) References cited:
WO-A1-2012/077756     JP-A- 2017 040 631
JP-A- 2017 223 604     JP-A- 2018 036 176
US-A1- 2018 209 965

• **RASTOGI VINAYAK ET AL: "Development and evaluation of realistic microbioassays in freely suspended droplets on a chip", vol. 1, no. 1, 1 March 2007 (2007-03-01), pages 14107 - 1, XP008149939, ISSN: 1932-1058, Retrieved from the Internet <URL:http://www.ncbi.nlm.nih.gov/pmc/journals /922/> [retrieved on 20070314], DOI: 10.1063/1.2714185**
• **SHAN CHEN ET AL: "AuNPs amplified magnetophoretic effect for colorimetric detection of nucleic acid", 10TH IEEE INTERNATIONAL CONFERENCE ON NANO/MICRO ENGINEERED AND MOLECULAR SYSTEMS, IEEE, 7 April 2015 (2015-04-07), pages 357 - 360, XP033170189, DOI: 10.1109/NEMS.2015.7147443**
• **BESSELINK G A J ET AL: "Signal amplification on planar and gel-type sensor surfaces in surface plasmon resonance-based detection of prostate-specific antigen", ANALYTICAL BIOCHEMISTRY, ACADEMIC PRESS, AMSTERDAM, NL, vol. 333, no. 1, 1 October 2004 (2004-10-01), pages 165 - 173, XP027195474, ISSN: 0003-2697, [retrieved on 20040909]**

- **RUILI PAN ET AL: "Gold nanoparticle-based enhanced lateral flow immunoassay for detection of Cronobacter sakazakii in powdered infant formula", JOURNAL OF DAIRY SCIENCE, vol. 101, no. 5, 1 May 2018 (2018-05-01), US, pages 3835 - 3843, XP055719734, ISSN: 0022-0302, DOI: 10.3168/jds.2017-14265**
- **JIN SEON-AH ET AL: "Gold decorated polystyrene particles for lateral flow immunodetection ofEscherichia coliO157:H7", MICROCHIMICA ACTA, SPRINGER VIENNA, VIENNA, vol. 184, no. 12, 5 October 2017 (2017-10-05), pages 4879 - 4886, XP036354916, ISSN: 0026-3672, [retrieved on 20171005], DOI: 10.1007/S00604-017-2524-5**

## Description

### Technical Field

The present invention relates to an immunochromatography that develops a solution comprising an analyte by utilizing a capillary phenomenon, to capture and detect a labeled analyte.

### Background Art

Immunochromatography generally utilizes a sandwich immunoassay method using a capturing antibody in a detection area and a detection antibody labeled with a particle such as a colloidal particle. Immunochromatographic method is performed as follows. When a liquid sample containing an analyte is developed and moved onto a test piece for an immunochromatographic method, a complex of labeled detection antibody : analyte (where ":" indicates binding) is formed. The liquid sample containing this complex develops and moves downstream because of a capillary phenomenon. The complex is captured by a capturing antibody immobilized on a detection region on the test piece for an immunochromatographic method. Finally, at the detection region, a complex of labeled detection antibody : analyte : capturing antibody (where ":" indicates binding) is formed, and the analyte is detected using, for example, line formation by a labeling substance as an index.

As the labeling substance, a metal colloid (for example, a gold colloid) that causes surface plasmon resonance, a colored nanoparticle (for example, a latex particle) , and other nanoparticles are used as labeling substances that can be visually (without apparatus) identified. Such colloidal particles do not require a special apparatus for observation, but if the concentration of the analyte in the sample solution is low, the signal intensity becomes low and may not visually recognizable.

A secondary sensitization method, such as a silver enhancement (see Patent Literature 1), has high sensitivity, but often increases operation steps or requires apparatus for observation.

In addition thereto, enhanced color intensity in coloring of a colloidal particle (see Patent Literature 2), composite particle formation from a metal colloid (see Patent Literatures 3 and 4), and higher sensitivity due to a core-shell structure (see Patent Literatures 5 and 6) have been reported, and the background tends to be high, for example when an unbound particle remains on the flow path, and a special material and step are required for particle production.

### Citation List

### Patent Literature

Patent Literature 1: JP Patent Publication (Kokai) No. 2008-286590 A
Patent Literature 2: WO2011/062157
Patent Literature 3: JP Patent Publication (Kokai) No. 2017-40631 A
Patent Literature 4: JP Patent Publication (Kokai) No. 2005-233744 A
Patent Literature 5: JP Patent Publication (Kokai) No. 2009-281760 A
Patent Literature 6: JP Patent Publication (Kokai) No. 2018-36176 A

Further prior art is known from:

RASTOGI VINAYAK ET AL: "Development and evaluation of realistic microbioassays in freely suspended droplets on a chip", BIOMICROFLUIDICS, AIP, US, vol. 1, no. 1, 1 March 2007 (2007-03-01), pages 14107-1, XP008149939, ISSN: 1932-1058, DOI: 10.1063/1.2714185;

SHAN CHEN ET AL: "AuNPs amplified magnetophoretic effect for colorimetric detection of nucleic acid", 10TH IEEE INTERNATIONAL CONFERENCE ON NANO/MICRO ENGINEERED AND MOLECULAR SYSTEMS, IEEE, 7 April 2015 (2015-04-07), pages 357-360, XP033170189, DOI: 10.1109/NEMS.2015.7147443;

BESSELINK G A J ET AL: "Signal amplification on planar and gel-type sensor surfaces in surface plasmon resonance-based detection of prostate-specific antigen",

ANALYTICAL BIOCHEMISTRY, ACADEMIC PRESS, AMSTERDAM, NL, vol. 333, no. 1, 1 October 2004 (2004-10-01), pages 165-173, P027195474, ISSN: 0003-2697;

Ruili Pan ET AL: "Gold nanoparticle-based enhanced lateral flow immunoassay for detection of Cronobacter sakazakii in powdered infant formula", JOURNAL OF DAIRY SCIENCE, vol. 101, no. 5, 1 May 2018 (2018-05-01), pages 3835-3843, XP055719734, US, ISSN: 0022-0302, DOI: 10.3168/jds.2017-14265;

JIN SEON-AH ET AL: "Gold decorated polystyrene particles for lateral flow immunodetection of Escherichia coli0157:H7", MICROCHIMICA ACTA, SPRINGER VIENNA, VIENNA, vol. 184, no. 12, 5 October 2017 (2017-10-05), pages 4879-4886, XP036354916, ISSN: 0026-3672, DOI: 10.1007/300604-017-2524-5; and

US patent publication US 2018/209965 A1.

## Summary of Invention

### Technical Problem

An object of the present invention is to achieve higher sensitivity for detection of an analyte by using a carrier particle that can be produced in a conventional step, with ease of detection and without increasing the background, in an immunochromatographic method.

### Solution to Problem

The absorption of light near a specific wavelength by surface plasmon resonance appears due to the collective vibration of surface electrons of resonant particles.

The present inventors consider that in an immunochromatographic method, when the resonant particle used for labeling is locally concentrated, the absorption of light near a specific wavelength increases and the signal is sensitized, and the present inventors have diligently studied a signal sensitization method. The present inventors have found that signal sensitization can be realized by a simple procedure by using two particles, one using a gold colloidal particle produced by a conventional method as a resonant particle and the other using a latex particle as a carrier particle, binding the gold colloidal particle to the carrier particle, and accumulating them, and have completed the present invention.

That is, the present invention with the feature of claim 1 is as follows.

[1] An immunochromatographic method using a resonant particle that causes surface plasmon resonance, and a carrier particle that causes no surface plasmon resonance and is larger in size than the resonant particle, each on which an affinity substance that binds to an analyte in a specimen sample is immobilized, the method comprising forming a complex represented by affinity substance bound carrier particle that binds to the analyte : analyte : affinity substance bound resonant particle that binds to the analyte (where ":" indicates binding) to thereby accumulate the resonant particle on the carrier particle, capturing the complex at a detection region on a test piece for an immunochromatographic method on which the affinity substance that binds to an analyte is immobilized as a capture substance, and detecting signals from the resonant particle due to surface plasmon resonance and a signal from the carrier particle to thereby detect the analyte.

Further aspects of the invention are:

[2] The immunochromatographic method according to [1], wherein the resonant particle is a gold colloidal particle.

[3] The immunochromatographic method according to [1] or [2], wherein the carrier particle is a latex particle.

[4] The immunochromatographic method according to any of [1] to [3], wherein the method uses a carrier particle having the resonant particle bound to a surface thereof in advance.

[5] The immunochromatographic method according to any of [1] to [3], wherein the method uses a carrier particle having the resonant particle encapsulated therein in advance.

[6] The immunochromatographic method according to any of [1] to [5], wherein the affinity substance bound to the resonant particle and the affinity substance bound to the carrier particle bind to different regions of the analyte.

[7] The immunochromatographic method according to any of [1] to [6], wherein the number of the resonant particle is 12 times or more the number of the carrier particle.

[8] The immunochromatographic method according to [7], wherein the number of the resonant particle is X times or more the number of the carrier particle [where X is represented by $(3(R + r)^2)/r^2$ with a carrier particle diameter = R and a resonant particle diameter = r, provided that the particle diameters are each a median value when measured in all directions].

[9] The immunochromatographic method according to any of [1] to [8], wherein the carrier particle and the analyte are first added to the test piece for an immunochromatographic method, and then the resonant particle is added.

[10] The immunochromatographic method according to any of [1] to [8], wherein the resonant particle and the carrier particle are mixed with the analyte and the resulting mixture is added to the test piece for an immunochromatographic method.

[11] The immunochromatographic method according to any of [1] to [8], wherein the resonant particle and the carrier particle are contained in a labeling region of the test piece for an immunochromatographic method.

[12] The immunochromatographic method according to any of [1] to [11], wherein the carrier particle is colored or labeled with a dye.

[13] The immunochromatographic method according to [12], wherein a color of the dye of the carrier particle is a color similar to a color (non-absorption peak) of the resonant particle.

[14] The immunochromatographic method according to any of [1] to [11], wherein the carrier particle comprises a fluorescent dye in the particle.

[15] The immunochromatographic method according to any of [1] to [11], wherein a fluorescent dye is bound to a surface of the carrier particle.

[16] The immunochromatographic method according to any of [1] to [15], wherein the analyte is an antigen or an antibody present in a living body.

[17] The immunochromatographic method according to any of [1] to [15], wherein the analyte is a pathogenic microorganism and a part thereof.

[18] An immunochromatographic test device as defined in independent claim 10.

[19] The test device according to [18], wherein the resonant particle is a gold colloidal particle.

[20] The test device according to [18] or [19], wherein the carrier particle is a latex particle.

[21] The test device according to any of [18] to [20], wherein the test device uses a carrier particle having the resonant particle bounded to a surface thereof in advance.

[22] The test device according to any of [18] to [20], wherein the test device uses a carrier particle having the resonant particle encapsulated therein in advance.

[23] The test device according to any of [18] to [22], wherein the affinity substance bound to the resonant particle and the affinity substance bound to the carrier particle bind to different regions of the analyte.

[24] The test device according to any of [18] to [23], wherein the number of the resonant particle is 12 times or more the number of the carrier particle.

[25] The test device according to [24], wherein the number of the resonant particle is X times or more the number of the carrier particle [where X is represented by $(3(R + r)^2)/r^2$ with a carrier particle diameter = R and a resonant particle diameter = r, provided that the particle diameters are each a median value when measured in all directions].

Advantageous Effects of Invention

The present invention realizes an increase in signal-to-noise ratio and higher sensitivity for detection of an analyte by an immunochromatographic method. At this time, a material production step and a complicated inspection operation technique that exceed the conventional techniques are not required. In addition, this phenomenon occurs only in the presence of an analyte, and thus the background is not enhanced by the mechanism of the present invention. In the method of the present invention, a resonant particle (or a part thereof) such as a gold colloidal particle is captured and accumulated on the surface of a limited number of carrier particle such as a latex particle, and thereby higher sensitivity is realized than when only the resonant particle is bound in a state of a low degree of accumulation in a wide range of a detection region where a capture substance (a substance having an affinity for an analyte, such as an antibody or an antigen) is present, on a test piece for an immunochromatographic method.

Brief Description of Drawings

[Figure 1] Figure 1 is a drawing illustrating a schematic diagram of a complex formation model (1) at a detection region.

[Figure 2] Figure 2 is a drawing illustrating a schematic diagram of a complex formation model (2) at a detection region.

[Figure 3] Figure 3 is a drawing illustrating the relationship between the number of carrier particle / number of resonant particle ratio and a signal peak area.

[Figure 4] Figure 4 is a drawing showing that a carrier particle and a resonant particle are bound via an analyte.

[Figure 5] Figure 5 is a drawing showing that a detection signal is increased by binding a resonant particle to a carrier particle via an analyte.

Description of Embodiments

Hereinafter, the present invention will be described in detail.

The present invention is an immunochromatographic method characterized by a labeling particle used.

In an immunochromatographic method including a lateral flow immunoassay, a test piece on which an affinity substance that specifically recognizes and binds to an analyte is immobilized and an insoluble carrier particle to which an affinity substance that specifically recognizes the analyte are bound are used. That is, the affinity substance is labeled with the insoluble carrier particle and used. The insoluble carrier particle binds to the analyte, develops and moves in a test piece having a porous structure by a capillary phenomenon, and binds to a capture substance that is an affinity substance that specifically binds to the analyte immobilized on a detection region in the test piece, and thereby, a complex of capture substance : analyte: insoluble carrier particle (where ":" indicates binding) is formed at the detection region, and the aggregated insoluble carrier particle is accumulated. By utilizing this, the light emitted at the region wherein the insoluble carrier particle is accumulated is measured as detection light using a detection apparatus or visually to detect the presence or absence of the accumulation of the insoluble carrier particle and analyze the presence or absence of the analyte.

Herein, an affinity substance that binds to an analyte may be simply referred to as an affinity substance.

As a labeling substance for labeling an affinity substance that binds to an analyte, a metal nanoparticle that causes surface plasmon resonance or a nanoparticle using an organic material such as a colored latex particle have been widely used.

In the present invention, two different particles are used each as an insoluble carrier particle which is a labeling substance. The two particles are a resonant particle and a carrier particle. The resonant particle is a particle that causes surface plasmon resonance, a carrier particle is a particle larger in diameter than the resonant particle, and the resonant particle binds to the surface of the carrier particle.

1. Carrier particle and resonant particle

As the resonant particle and the carrier particle, those having an affinity substance that binds to an analyte immobilized on the surface thereof are used. The affinity substance is a substance that binds to an analyte, and if the analyte is an antibody, the affinity substance is an antigen to which the antibody binds, and if the analyte is an antigen, the affinity substance is an antibody that binds to the antigen. The analyte and the affinity substance are not limited to the combination of the antigen and the antibody, and for example, the analyte and the affinity substance may have a ligand-receptor or receptor-ligand relationship. Examples of such an affinity substance include polypeptides and other compounds that can bind to the analyte.

As a method of immobilizing an affinity substance that binds to an analyte on the surface of the resonant particle or the carrier particle, physical binding, electrical binding, chemical binding, or the like may be used. Examples thereof include immobilization utilizing covalent binding between functional groups and immobilization by physical adsorption. After immobilizing an affinity substance on the resonant particle or the carrier particle, in order to suppress non-specific binding of another substance to these particles, the particle surface may be blocked using a high molecular weight polymer such as polyethylene glycol, a protein such as bovine serum albumin (BSA) or milk casein, or the like. Herein, a carrier particle to which an affinity substance that binds to an analyte is bound and a resonant particle to which an affinity substance that binds to an analyte is bound are sometimes referred to as an affinity substance bound carrier particle (a carrier particle to which an affinity substance is bound) and an affinity substance bound resonant particle, respectively.

The resonant particle may be any particle as long as it is a particle absorbing light near a specific wavelength by causing surface plasmon resonance. Examples thereof include a metal colloid, and if the resonant particle is a metal colloid, the type and the shape of a metal, an alloy, a metal oxide, a metal compound, or the like are not limited. The diameter of the resonant particle is 10 to 200 nm, preferably 10 to 100 nm, and further preferably 10 to 50 nm. The metal colloid also includes a core-shell type wherein one gold colloidal particle is coated with monolayer silica or the like. The resonant particle does not need to be a single metal nanoparticle, and may be a combination thereof. Preferably, the color tone due to such a combination is advantageous to the observer, or the wavelength of light that is absorbed / not absorbed is advantageous to the observation equipment.

The carrier particle is larger in size than the resonant particle, but the material, the shape, and the like thereof are not limited. The diameter of the carrier particle is tens of nm to several hundreds of nm, preferably 50 nm to 800 nm, and further preferably 200 to 600 nm. Examples of the carrier particle include a colored latex particle and a latex particle containing a fluorescent dye. The latex particle is a particle that forms an emulsion by being dispersed in water in colloidal form. The material of the particle is not limited, and a material used as a material to which a protein such as an antibody, an antigen, a ligand, or a receptor is bound to be coated in technical fields of a test drug and the like can be used. Examples of the material include polystyrene, a styrene copolymer such as a styrene-acrylic acid copolymer, a resin such as polycarbonate, polymethylene methacrylate (PMMA), or polyvinyl toluene, silica, and cellulose. Among these, a styrene-based particle is preferable. The styrene-based particle refers to a particle made of polystyrene or a copolymer of styrene or a styrene derivative with a polymerizable unsaturated carboxylic acid, a polymerizable unsaturated sulfonic acid, or the like. Examples of the styrene derivative include chloromethylstyrene and divinylbenzene, examples of the polymerizable unsaturated carboxylic acid include acrylic acid and methacrylic acid, and examples of the polymerizable unsaturated sulfonic acid include sodium styrenesulfonate. In the present invention, a styrene-based latex particle is referred to as a polystyrene latex particle. In addition, a carrier particle that exhibits a color similar to that of a resonant particle or interacts with a resonant particle to increase the fluorescence intensity is preferable. In addition, in order to achieve these, carrier particles having a plurality of materials, shapes, and sizes can be used. When coloring the carrier particle with a dye, the detection sensitivity may be increased by using a non-absorption peak (wavelength outside the absorption peak) of the resonant particle or a hue recognized as a color similar to that of the resonant particle. When the carrier particle contains a fluorescent dye, the carrier particle may be a carrier particle containing a fluorescent dye having an excitation wavelength or a fluorescence wavelength similar to the non-absorption peak of the resonant particle, or a carrier particle having a fluorescent particle having such a fluorescence wavelength bound thereto. In addition, the carrier particle may be a combination of a resonant particle that causes surface enhanced fluorescence (SEF, also referred to as metal enhanced fluorescence or plasmon enhanced fluorescence) and a carrier particle containing a fluorescent dye or a carrier particle having a fluorescent particle bound thereto. When the carrier particle contains a fluorescent dye, the fluorescence can be detected using a fluorescence detection apparatus.

In the immunochromatographic method of the present invention, finally, the resonant particle is bound to the surface of the carrier particle. Preferably, the resonant particle is bound to the surface of the carrier particle in such a way as to cover the entire carrier particle surface.

The resonant particle may be bound to a part of the surface of the carrier particle in advance. For the binding of the resonant particle to the carrier particle, physical binding, electrical binding, chemical binding, or the like may be used. Examples thereof include binding utilizing covalent binding between functional groups and binding by physical adsorption. In addition, the resonant particle may be included inside the carrier particle in advance. Including the resonant particle inside the carrier particle is also referred to as encapsulating the resonant particle in the carrier particle. In order to encapsulate the resonant particle in the carrier particle, for example, a core-shell particle may be used as the carrier particle. The core-shell particle refers to a particle wherein a porous layer is present as a shell on the surface of a non-porous core particle, and a resonant particle can be present in a pore of the porous layer. The type of the core-shell particle is not limited, and for example, a commercially available core-shell particle can be used.

Examples of an existing technique for including a resonant particle inside or on the surface of a carrier particle include techniques for including a particle that causes surface plasmon resonance inside a detection particle (a particle on which a detection antibody is immobilized) made of silica or a resin as a material (JP Patent Publication (Kokai) No. 2009-281760 A and JP Patent Publication (Kokai) No. 2018-36176 A), and techniques for modifying the surface of a detected particle with a particle that causes surface plasmon resonance (JP Patent Publication (Kokai) No. 2017-40631 A and JP Patent Publication (Kokai) No. 2005-233744 A). According to descriptions in these literatures, a carrier particle having a resonant particle included inside or on the surface of the same can be manufactured. Furthermore, the resonant particle and the carrier particle may be bound to each other via an analyte. That is, an affinity substance that binds to the analyte bound to the resonant particle and an affinity substance that binds to the analyte bound to the carrier particle binds to each other via the analyte, and thereby the resonant particle binds to the surface of the carrier particle. At this time, a complex represented by affinity substance bound carrier particle: analyte : affinity substance bound resonant particle (where ":" indicates binding) is formed.

In the immunochromatographic method, a specimen that is liquid flows on a test piece while dissolving a reagent, and develops and moves; and at this time, the above-mentioned complex is formed while the affinity substance bound carrier particle and the affinity substance bound resonant particle develop on the test piece. Finally, the complex is captured and accumulated by the affinity substance that binds to the analyte immobilized on a detection region on an test piece for an immunochromatographic method, and by the absorption of light near a specific wavelength by surface plasmon resonance at the detection region, a signal is emitted from the resonant particle and a signal is emitted from the carrier particle; and thus the total signals including the signal due to surface plasmon resonance and the signal from the carrier particle can be detected. At this time, a carrier particle not having the resonant particle included inside or on the surface of the same may be used to bind the carrier particle and the resonant particle in the developing solution, or a carrier particle having the carrier particle inside or on the surface of the same may be used to further bind the resonant particle to the carrier particle in the developing solution. By this method, the density of the resonant particle on the surface of the carrier particle is increased, the surface plasmon resonance is sensitized, and higher sensitivity wherein the signal-to-noise ratio is higher than that of the conventional technique can be achieved.

As an affinity substance that binds to an analyte, there are at least three affinity substances: an affinity substance that binds to the resonant particle and an affinity substance that binds to the carrier particle as well as an affinity substance that is immobilized on a detection region on a test piece. Each of these affinity substances (groups) may be one type or a plurality of types. In addition, the respective compositions of the affinity substances or affinity substance groups may be the same or be partially or all different. Here, the statement that the affinity substances are the same or common means that the affinity substances bind to the same region of the same analyte, and the statement that the affinity substances are different or not common means that the affinity substances bind to the same analyte but bind to different regions of the analyte. The statement that the composition of the affinity substance group is the same means that all the affinity substances used are common regardless of the amount and proportion of each affinity substance. A partially different composition refers to including at least one non-common affinity substance and at least one common affinity substance. An all-different composition refers to including no common affinity substance. For example, if the affinity substance is an antibody against the analyte, when the statement that the affinity substance bound to the resonant particle and the affinity substance bound to the carrier particle are the same or common, both the affinity substances recognize and bind to the same epitope of the analyte that is an antigen. On the other hand, when both the affinity substances are different or not common, both the affinity substances recognize different epitopes of the analyte that is an antigen. When the analyte is a large molecule such as a virus particle or a polymer, there are a plurality of regions to which the affinity substance binds in the same analyte, and thus by a common affinity substance on the resonant particle and/or on the carrier particle and/or on a detection region on a test piece, a complex of the resonant particle and the carrier particle can be formed at a detection region on a test piece for an immunochromatographic method. On the other hand, when the analyte is a relatively small molecule, such a complex is difficult to form. This is because the number of regions to which the affinity substance binds in the same analyte is small, and the common affinity substances on the particles or detection regions compete with each other for binding to the analyte. Therefore, the affinity substance of the resonant particle, the affinity substance of the carrier particle, and the affinity substance of the detection region on the test piece for an immunochromatographic method are preferably different substances or all have different compositions.

There is a preferable range for the ratio of the number of a resonant particle to the number of a carrier particle. The case where the maximum number of a resonant particle bind to a carrier particle, that is, the case where the number of carrier particle / number of resonant particle ratio is the minimum, will be discussed on the presumption that these particles are spheres. If the reaction time is infinite, the sizes of the resonant particle and the carrier particle are similar, and the distance between the particle surfaces and the size of the analyte are negligible relative to the above sizes, the number of the resonant particle that can bind to the carrier particle is considered to be 12 from the three-dimensional kissing number. Because of this, the optimum value of the number of carrier particle / number of resonant particle ratio practiced in the present invention is, in many cases, 1/12 or less in order to maximize surface plasmon resonance. Here, the three-dimensional kissing number refers to the maximum number of unit spheres that can be arranged when they are arranged around a three-dimensional unit sphere in such a way as to touch the same without any intersections. That is, the number of a resonant particle is preferably 12 times or more the number of a carrier particle. This multiple is considered to be affected by the reaction time, the resonant particle / carrier particle diameter ratio, the size of the analyte, the concentration of the analyte, and the like.

A generalized optimum value will be discussed according to the particle diameter. Assuming that a maximum number of spheres B of a radius r is in contact with a sphere A of a radius R (R ≥ r), the maximum number of contact particles of B is approximately estimated from the surface area of the sphere formed by the center of B and the maximum cross-sectional area of B; and when the filling rate (a maximum of about 0.75 for a three-dimensional sphere and a maximum of about 0.9 for a two-dimensional circle; in this approximation method, the larger R is than r, the closer the rate is to 0.9) is defined as a, the maximum number X of contact particles of the sphere B in contact with the sphere A can be approximated as follows.

$$X = 4a(r+R)^2/r^2$$

This approximation can be used to calculate the maximum number of contact particles between every two of antigen particles, resonant particles, and carrier particles, and is affected by the reaction time and the analyte concentration, but may be useful for estimating an optimum value of the number of carrier particle / number of resonant particle ratio.

With the carrier particle diameter = R and the resonant particle diameter = r, the number of the resonant particle is preferably $(4a(R + r)^2)/r^2$ times the number of the carrier particle. With a = 0.75, the number of the resonant particle is preferably $(3(R + r)^2)/r^2$ times the number of the carrier particle.

2. Analyte and specimen

Main examples of the analyte include a marker in the living body and a pathogenic microorganism. The marker in the living body refers to a substance such as a protein, a low molecular weight compound, a lipid, or a sugar for detecting a specific disease. As the specimen, mainly an aqueous phase specimen or a diluted solution of a specimen obtained by dilution with a buffer can be used. Examples of the specimen include serum, plasma, blood, urine, saliva, tissue fluid, spinal fluid, pharyngeal or nasal swab fluid, pharyngeal or nasal lavage fluid, a body fluid such as nasal aspirate, feces, fecal suspension, and culture fluid. The size of the analyte is not limited, and it is expected that the effect of increasing the detection sensitivity according to the present invention will be small when the size of the analyte is remarkably larger than that of the carrier particle. In addition, when the size of the carrier particle is smaller than that of the resonant particle, it is expected that the effect of increasing the detection sensitivity according to the present invention will be small. Because of these, it is preferable that the size of the resonant particle < the size of the analyte < the size of the carrier particle, or the size of the analyte < the size of the resonant particle < the size of the carrier particle.

3. Immunochromatographic method

The immunochromatographic method can be performed by a known method using a known device for an immunochromatographic method. The device for an immunochromatographic method is also referred to as a test piece (strip) for an immunochromatographic method, and is made of a porous water-absorbent material made of for example a material consisting of nitrocellulose, cellulose acetate, nylon, polyether sulfone, polyvinyl alcohol, polyester, glass fiber, polyolefin, cellulose, or a mixed fiber thereof, through which a specimen sample liquid can move due to a capillary phenomenon. The thickness of a membrane is not particularly limited, and is preferably about 100 to 200 μm. In addition, the membrane is used as a rectangular strip, and the size thereof is not particularly limited, and usually, the short side × long side is about 3 mm to 9 mm × 40 mm to 60 mm. The test piece for an immunochromatographic method has a detection region, a sample addition region, an absorption band, and the like, and may have a labeling region. On the detection region, an affinity substance that can specifically bind to an analyte to be detected and capture the analyte is immobilized. The detection region is sometimes referred to as the capture region. Herein, an affinity substance immobilized on the detection region of a test piece for an immunochromatographic method is sometimes referred to as a detection region immobilized affinity substance. The sample addition region is a region to which the specimen sample liquid is added, and the specimen sample liquid may be directly added onto the test piece for an immunochromatographic method, but a pad made of a water-absorbent material, for example a sponge or a non-woven fabric such as glass fiber, may be provided on the test piece and added to that portion. The pad at the sample addition region is referred to as the sample pad. The absorption band, also referred to as an absorption pad region, is provided at one end different from the sample addition region of the test piece for an immunochromatographic method of the present invention, and, after adding to the sample addition region, absorbs the sample liquid that develops and moves on the test piece, and thereby promotes the flow of the sample liquid on the test piece. The absorption band is made of a water-absorbent material so that a large amount of liquid can be absorbed; and for example, a non-woven fabric made of cellulose, glass fiber, or the like is used. In addition, the size thereof is not particularly limited, and usually, the short side × long side is 3 mm to 15 mm × 10 mm to 40 mm, and the thickness is about 0.5 mm to 3 mm. The labeling region is a region containing an affinity substance bound carrier particle and/or an affinity substance bound resonant particle. For the labeling region, a water-absorbent material such as a sponge and a non-woven fabric made of glass fiber or the like is used, and the size thereof is not particularly limited, and usually, the short side × long side is 3 mm to 10 mm × 3 mm to 10 mm and the thickness is about 0.5 mm to 3 mm, and an affinity substance bound carrier particle and/or an affinity substance bound resonant particle is contained in a dry state. When the labeling region is used by containing an affinity substance bound carrier particle and/or an affinity substance bound resonant particle in the above-mentioned water-absorbent material, the labeling region can be referred to as a conjugate pad. The affinity substance bound carrier particle and the affinity substance bound resonant particle may be contained in the same labeling region or contained in different labeling regions.

In addition, the affinity substance bound carrier particle and the affinity substance bound resonant particle do not necessarily have to be contained on the test piece for an immunochromatographic method. In this case, there is no labeling region on the test piece. In addition, only one of the affinity substance bound carrier particle and the affinity substance bound resonant particle may be contained on the test piece. For the labeling region, the above-mentioned water-absorbent material may be impregnated with the affinity substance bound carrier particle and/or the affinity substance bound resonant particle and dried. When the sample addition region side is upstream and the absorption band side is downstream when the specimen sample liquid flows from the sample addition region to the absorption band on the test piece, the labeling region may be provided downstream of the sample addition region and upstream of the detection region. The test piece for an immunochromatographic method may further have a control display region. The control display region is a region indicating that the test has been performed accurately. For example, the control display region is present downstream of the detection region, and when a specimen sample passes through the detection region and reaches the control display region, a signal is emitted by means of coloring or the like. On the control display unit, a substance that binds to an affinity substance that binds to an analyte bound to a particle, for example, an antibody, may be immobilized, or a reagent such as a pH indicator that changes its color when the specimen sample arrives may be immobilized.

The immunochromatographic method of the present invention detects an analyte in the following steps.

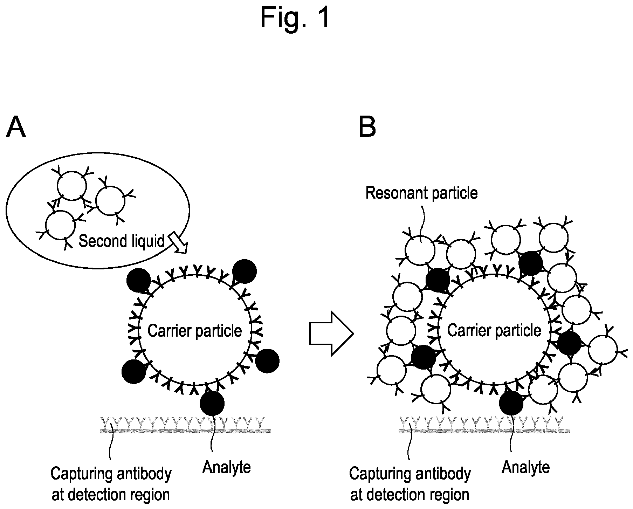

In the immunochromatographic method of the present invention, a complex is finally formed at the detection region, the complex being represented by detection region immobilized affinity substance : analyte : <affinity substance bound carrier particle : analyte : affinity substance bound resonant particle> (where ":" indicates binding; the portion enclosed by "<>" refers to a state in which either the affinity substance bound carrier particle or the affinity substance bound resonant particle and the analyte are alternately bound so that these three substances are bound in branched form; for example, it is a state of a complex of the carrier particle, the resonant particle, and the substance under test as shown in Figure 1B; the analyte bound to the detection region on which an affinity substance is immobilized and the analyte represented by <affinity substance bound carrier particle : analyte : affinity substance bound resonant particle> may be the same or different) (herein, sometimes referred to as the "analyte particle complex captured at the detection region"). Surface plasmon resonance at the detection region increases the absorption of light near a specific wavelength by the resonant particle. The above absorption of light near a specific wavelength by the resonant particle appears as visual color change, and otherwise as a (non-)absorption peak wavelength, and the analyte can be detected by measuring these visually or with a detection apparatus. At this time, in order to obtain a high signal, the minimum unit of an analyte and particle complex (a complex of an analyte and a particle) captured at the detection region is preferably a complex wherein the maximum number of resonant particles that can bind to the carrier particle surface via the analyte are bound; and this complex needs to be bound to the affinity substance immobilized on the detection region via the analyte.

For example, in an immunochromatographic method of detecting an analyte in a specimen sample, by adding the specimen sample to the sample addition region on a test piece, the affinity substance bound carrier particle and the affinity substance bound resonant particle are developed and moved on the test piece along with the analyte by a capillary phenomenon. At this time, (1) it is possible to develop and move the affinity substance bound carrier particle earlier than the affinity substance bound resonant particle to form a complex represented by detection region immobilized affinity substance : analyte : affinity substance bound carrier particle (where ":" indicates binding) at the detection region, and then develop and move the affinity substance bound resonant particle to form an analyte and particle complex at the detection region to detect a signal from the resonant particle due to surface plasmon resonance. Alternatively, (2) it is possible to simultaneously develop and move the affinity substance bound carrier particle and the affinity substance bound resonant particle, and, while forming a complex during the development and movement, the complex being represented by <affinity substance bound carrier particle : analyte : affinity substance bound resonant particle> (where ":" indicates binding; the portion enclosed by "<>" refers to a state in which either the affinity substance bound carrier particle or the affinity substance bound resonant particle and the analyte are alternately bound so that these three substances are bound in branched form; for example, it is a state of a complex of the carrier particle, the resonant particle, and the substance under test as shown in Figure 1B), finally form an analyte particle complex captured at the detection region to detect a signal from the resonant particle due to surface plasmon resonance.

In the case of (1), at the detection region of the test piece for an immunochromatographic method, a complex represented by detection region immobilized affinity substance : affinity substance bound carrier particle : analyte (where ":" indicates binding) is first formed, then the affinity substance bound resonant particle binds to the complex, and finally, an analyte and particle complex captured at the detection region is formed. In the case of (1), it is possible to first, mix the affinity substance bound carrier particle and the specimen, add the resulting mixture (first liquid) to the addition region of the test piece, and develop and move the mixture on the test piece for an immunochromatographic method, and then add a liquid containing the affinity substance bound resonant particle (second liquid) to the addition region of the test piece for an immunochromatographic method, and develop and move the liquid. Figure 1 illustrates a schematic diagram of complex formation of the model of (1). In Figure 1, an antibody is used as an affinity substance that is immobilized on the detection region of the test piece for an immunochromatographic method and as an affinity substance that binds to the carrier particle and the resonant particle. Figure 1A illustrates the reaction when the first solution is added, and a complex of antibody bound carrier particle : analyte: detection region capturing antibody (where ":" indicates binding) is formed at the detection region. Figure 1B illustrates the reaction when the second solution containing the antibody bound resonant particle is added, and the final complex is formed at the detection region. In Figure 1, the antibodies of the antibody bound carrier particle, the antibody bound resonant particle, and the detection region capturing antibody may be the same or different.

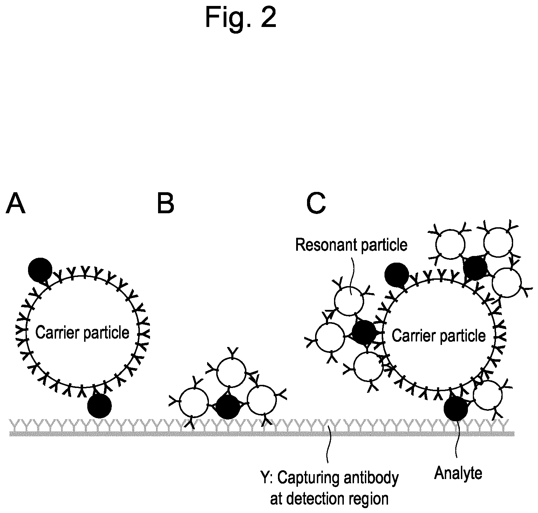

In the case of (2), the affinity substance bound carrier particle, the affinity substance bound resonant particle, and a specimen containing the analyte may be mixed to add the resulting mixture to the sample addition region of the test piece for an immunochromatographic method. Alternatively, the affinity substance bound carrier particle and the affinity substance bound resonant particle may be contained in the same labeling region or different labeling regions on the test piece for an immunochromatographic method to use the added specimen sample to develop and move the affinity substance bound carrier particle and the affinity substance bound resonant particle along with the analyte. Figure 2 illustrates a state of complex formation of the model of (2). During the development and movement, the complexes illustrated in Figures 2A, B, and C, or a complex to which these are further bound via an analyte is formed, and these complexes are captured at the detection region of the test piece for an immunochromatographic method. In the immunochromatographic method of the present invention, the complex illustrated in Figure 2C is preferable as the complex to be formed at the detection region of the test piece for an immunochromatographic method. In Figure 2, the antibodies of the antibody bound carrier particle, the antibody bound resonant particle, and the detection region capturing antibody may be the same or different.

A resonance material to which the affinity substance is not bound may be allowed to be present in advance inside or on a part of the surface of the affinity substance bound carrier particle, and further, the affinity substance bound resonant particle may be bound to that affinity substance bound carrier particle. In this case, in addition to the resonant particle contained in the affinity substance bound carrier particle in advance, the affinity substance bound resonant particle added to the test piece later binds further to the affinity substance bound carrier particle, and thereby the degree of accumulation of the resonant particle on the affinity substance bound carrier particle is increased, and thus the signal generated by surface plasmon resonance is more sensitized, and accordingly the analyte can be detected with higher sensitivity.

The developing solution for developing and moving a liquid and a reagent containing the affinity substance bound carrier particle and/or the affinity substance bound resonant particle preferably contains a polymer, a protein, a buffering agent, and/or a surfactant that suppress a non-specific reaction. The buffering agent to be contained is not limited as long as it has a buffering action that does not exert a fatal influence on detection due to addition of a sample, change in concentration, or contamination by foreign matter. A HEPES buffer, a phosphate buffer, an acetate buffer, a Tris-hydrochloric acid buffer, and the like are preferable, and a Tris-hydrochloric acid buffer is particularly preferable.

Disclosed but not claimed is a kit for an immunochromatographic method, which comprises a carrier particle to which an affinity substance for an analyte is bound and a resonant particle to which an affinity substance for an analyte is bound. The kit further includes a test piece for an immunochromatographic method, a brochure, and a buffer. The above-mentioned affinity substance bound carrier particle and affinity substance bound resonant particle may be contained as a reagent different from the test piece for an immunochromatographic method, or be contained in the labeling region of the test piece for an immunochromatographic method.

The present invention also includes a test piece for an immunochromatographic method, which is defined in accordance with independent claim 10.

Examples

The present invention will be specifically described with reference to the following Examples, but the present invention is not limited by these Examples.

<Example 1>

Test method

A gold colloidal particle having a diameter of 20 nm (BBI, UK) as a resonant particle and a latex particle having a diameter of 400 nm (JSR, Japan) as a carrier particle were purchased. An antibody against norovirus was immobilized on each particle.

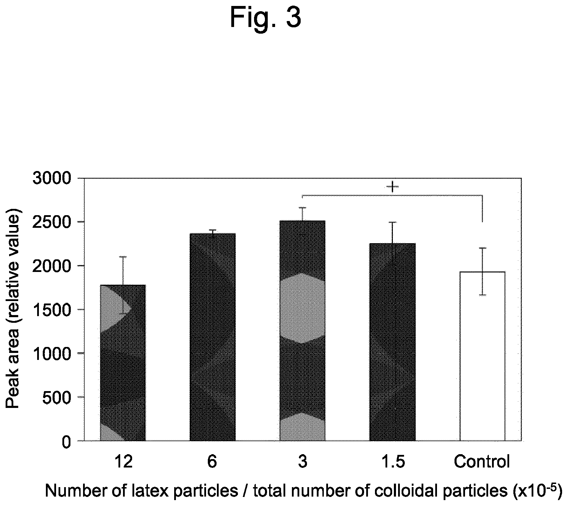

For the strip (white) for an immunochromatographic method, a capturing antibody to be bound to norovirus was immobilized on the detection region, and an anti-mouse antibody was immobilized in line form on the control display region downstream thereof as a positive control. A gold colloidal particle having a constant concentration, latex particles having various concentrations, and the analyte Norovirus VLP were mixed in a Tris buffer containing 1.5% Tx-100, a strip for an immunochromatographic method was immediately immersed, and the mixture was developed and moved on the strip for an immunochromatographic method. After 15 minutes, the sample pad (material member that contacts the solution) was cut, the detection area was image-scanned, the obtained image was analyzed using the image processing software ImageJ as shown in Figure 3, and a significant difference test of the obtained signal peak area value was performed using the statistical software R.

Test results

Figure 3 illustrates the peak area when the white latex particles having various concentrations were mixed with a constant gold colloid concentration. Each bar represents SD and the "+" indicates that there is a significant difference to the control (Dunnett's test; $p < 0.05$).

As illustrated in Figure 3, a significant increase in signal was observed when the number of carrier particle / number of resonant particle ratio was $3 \times 10^{-5}$.

Discussion

The white latex particles have the same color as the strip for an immunochromatographic method and do not directly contribute to the detection sensitivity in the present Example. Because of this, it is considered that the increase in sensitivity near the peak was caused by the concentration of the gold colloid on the white latex and the amplification of the effect of surface plasmon resonance.

On the left side of the peak value, a decrease in sensitivity is observed as compared with the optimum value, and this is when the proportion of the white latex particles is higher than the optimum value; and it is considered that the sensitivity decreased because of effects such as reduction of surface plasmon resonance, inhibition of binding of the gold colloid surface antibody due to steric hindrance, and physically hiding the gold colloid behind the white latex from the observation field.

The optimum value, number of the resonant particle / number of carrier particle ratio, is smaller than 1/12. The first reason for this is that the resonant particle / carrier particle diameter ratio is away from 1. If the maximum number X of the resonant particle that can contact the carrier particle is recalculated as a = 0.9, then $X = 4a(r + R)^2/r^2 = 1587$, and the optimum value of number of carrier particle / number of resonant particle ratio is 1/1587 or less; and the result of the present Example also satisfies it. It is considered that the reasons why the optimum ratio was further smaller than this in the result of the present Example include the virus particle diameter which is about twice the particle diameter of the gold colloidal particle, the limited antigen concentration, and the limited reaction time. From the above results, it was clarified that by mixing the resonant particle with the carrier particle at a specific number of resonant particle / number of carrier particle ratio, the sensitivity is higher than that with the resonant particle alone.

In addition, in the present Example, white latex was used in order to check the effect of surface plasmon resonance, and various combinations of a resonant particle and a colored or fluorescent dye labeled carrier particle can further increase the sensitivity.

<Example 2>

Test method

An immunochromatographic method was performed using a membrane on which streptavidin was immobilized in line form. The membrane was impregnated with a Tris solution containing 1.5% Tx-100 including a biotinylated influenza virus antibody coated latex particle (blue), an influenza virus antibody coated gold colloidal particle (red), and inactivated influenza virus. The negative control used had any of the latex particle, the gold colloidal particle, and the virus excluded.

Test results

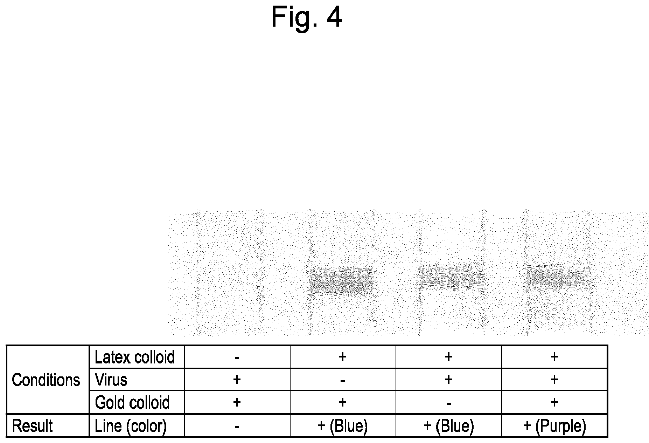

Figure 4 illustrates test results.
As illustrated in Figure 4, a purple line of a complex of streptavidin : biotinylated influenza virus antibody coated latex particle : inactivated influenza virus : influenza virus antibody coated gold colloidal particle (where : indicates binding) was observed. In the negative control, no colored line was observed, or a blue line was observed.

Discussion

It was suggested that the carrier particle and the resonant particle were bound to each other via the analyte.
It was suggested that the present invention can be practiced when influenza virus was used as the analyte.

<Example 3 >

Test method

An immunochromatographic method was performed using a membrane on which streptavidin was immobilized in line form. The membrane was impregnated with a Tris solution containing 1.5% Tx-100 including a biotinylated norovirus antibody coated latex particle (blue), a norovirus antibody coated gold colloidal particle (red), and a norovirus virus like particle. The negative control used had any of the latex particle, the gold colloidal particle, and the virus excluded.

Test results

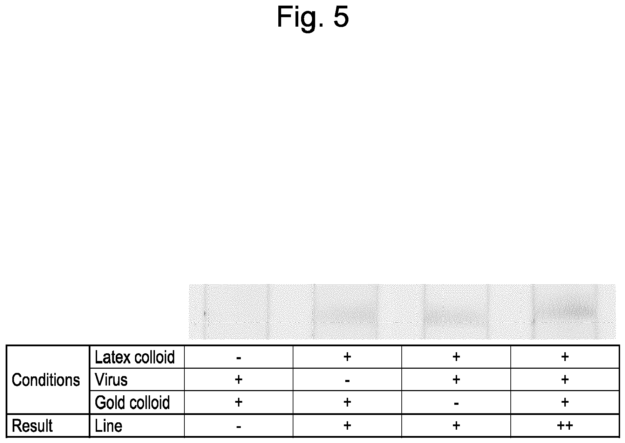

Figure 5 illustrates test results.
As illustrated in Figure 5, because of formation of a complex of streptavidin : biotinylated norovirus antibody coated latex particle : norovirus virus like particle : norovirus antibody coated gold colloidal particle (where : indicates binding), a higher signal was detected than when no complex was formed.

Discussion

It was suggested that the detection signal is enhanced by the binding of the resonant particle to the carrier particle via the analyte.

Industrial Applicability

The immunochromatographic method of the present invention can be used for highly sensitive detection of an analyte.

**Claims**

1. An immunochromatographic method using a resonant particle that causes surface plasmon resonance, and a carrier particle that causes no surface plasmon resonance and is larger in size than the resonant particle, each on which an affinity substance that binds to an analyte in a specimen sample is immobilized, the method comprising forming a complex represented by affinity substance bound carrier particle that binds to the analyte : analyte : affinity substance bound resonant particle that binds to the analyte (where ":" indicates binding) to thereby accumulate the resonant particle on the carrier particle, capturing the complex at a detection region on a test piece for an immunochromatographic method on which the affinity substance that binds to an analyte is immobilized as a capture substance, and detecting signals from the resonant particle due to surface plasmon resonance and a signal from the carrier particle to thereby detect the analyte.

2. The immunochromatographic method according to claim 1, wherein the resonant particle is a gold colloidal particle, or the carrier particle is a latex particle.

3. The immunochromatographic method according to any claim 1 or 2, wherein the method uses a carrier particle having the resonant particle bound to a surface thereof in advance, or a carrier particle having the resonant particle encapsulated therein in advance.

4. The immunochromatographic method according to any one of claims 1 to 3, wherein the affinity substance bound to the resonant particle and the affinity substance bound to the carrier particle bind to different regions of the analyte.

5. The immunochromatographic method according to any one of claims 1 to 4, wherein the number of the resonant particle is 12 times or more the number of the carrier particle, or the number of the resonant particle is X times or more the number of the carrier particle [where X is represented by $(3(R + r)^2)/r^2$ with a carrier particle diameter = R and a resonant particle diameter = r, provided that the particle diameters are each a median value when measured in all directions].

6. The immunochromatographic method according to any one of claims 1 to 5, wherein (i) the carrier particle and the analyte are first added to the test piece for an immunochromatographic method, and then the resonant particle is added, (ii) the resonant particle and the carrier particle are mixed with the analyte and the resulting mixture is added to the test piece for an immunochromatographic method, or the resonant particle and the carrier particle are contained in a labeling region of the test piece for an immunochromatographic method.

7. The immunochromatographic method according to any one of claims 1 to 6, wherein the carrier particle is colored or labeled with a dye, or a color of the dye of the carrier particle is a color similar to a color (nonabsorption peak) of the resonant particle.

8. The immunochromatographic method according to any one of claims 1 to 7, wherein the carrier particle comprises a fluorescent dye in the particle, or a fluorescent dye is bound to a surface of the carrier particle.

9. The immunochromatographic method according to any one of claims 1 to 8, wherein the analyte is an antigen or an antibody present in a living body, or the analyte is a pathogenic microorganism and a part thereof.

10. An immunochromatographic test device comprising in a labeling region the resonant and carrier particles as specified in claim 1, wherein the device is configured to perform the following steps upon flowing the sample on said device:

the complex forming step as specified in claim 1,
the complex capturing step as specified in claim 1, and
providing the detectable signals as specified in claim 1.

11. The immunochromatographic test device according to claim 10, wherein the resonant particle is a gold colloidal particle, or the carrier particle is a latex particle.

12. The immunochromatographic test device according to claim 10 or 11, wherein the test piece uses a carrier particle having the resonant particle bounded to a surface thereof in advance, or the test piece uses a carrier particle having the resonant particle encapsulated therein in advance.

13. The immunochromatographic test device according to any one of claims 11 to 12, wherein the affinity substance bound to the resonant particle and the affinity substance bound to the carrier particle bind to different regions of the analyte.

14. The immunochromatographic test device according to any one of claims 11 to 13, wherein the number of the resonant particle is 12 times or more the number of the carrier particle, or the number of the resonant particle is X times or more the number of the carrier particle [where X is represented by $(3(R + r)^2)/r^2$ with a carrier particle diameter = R and a resonant particle diameter = r, provided that the particle diameters are each a median value when measured in all directions].

**Patentansprüche**

1. Immunochromatographisches Verfahren unter Verwendung eines Resonanzpartikels, das Oberflächenplasmonenresonanz hervorruft, und eines Trägerpartikels, das keine Oberflächenplasmonenresonanz hervorruft und größer ist als das Resonanzpartikel, auf denen jeweils eine Affinitätssubstanz immobilisiert ist, die sich an einen Analyt in einer Probe bindet, wobei das Verfahren die Bildung eines Komplexes, der durch ein an eine Affinitätssubstanz gebundenes Trägerpartikel dargestellt wird, das sich an das an eine Analyt:Analyt:Affinitätssubstanz gebundene Resonanzpartikel bindet, das sich an den Analyt bindet (wobei ‚:‛ Bindung bedeutet), um dadurch das Resonanzpartikel auf dem Trägerpartikel zu akkumulieren, das Einfangen des Komplexes in einem Nachweisbereich auf einem Teststück für ein immunochromatographisches Verfahren, auf dem die Affinitätssubstanz, die sich an einen Analyt bindet, als Einfangsubstanz immobilisiert ist, und das Erfassen von Signalen von dem Resonanzpartikel aufgrund von Oberflächenplasmonenresonanz und eines Signals von dem Trägerpartikel, um dadurch den Analyt zu erfassen, umfasst.

2. Immunochromatographisches Verfahren nach Anspruch 1, wobei das Resonanzpartikel ein kolloidales Goldpartikel ist oder das Trägerpartikel ein Latexpartikel ist.

3. Immunochromatographisches Verfahren nach einem der Ansprüche 1 oder 2, wobei das Verfahren ein Trägerpartikel verwendet, an dessen Oberfläche das Resonanzpartikel im Voraus gebunden ist, oder ein Trägerpartikel, in dem das Resonanzpartikel im Voraus eingekapselt ist.

4. Immunochromatographisches Verfahren nach einem der Ansprüche 1 bis 3, wobei die an das Resonanzpartikel gebundene Affinitätssubstanz und die an das Trägerpartikel gebundene Affinitätssubstanz sich an unterschiedliche Bereiche des Analyten binden.

5. Immunochromatographisches Verfahren nach einem der Ansprüche 1 bis 4, wobei die Anzahl der Resonanzpartikel das 12-fache oder mehr der Anzahl der Trägerpartikel beträgt oder die Anzahl der Resonanzpartikel das X-fache oder mehr der Anzahl der Trägerpartikel beträgt [wobei X durch $(3(R + r)^2)/r^2$ mit einem Trägerpartikeldurchmesser = R und einem Resonanzpartikeldurchmesser = r dargestellt wird, mit der Maßgabe, dass die Partikeldurchmesser jeweils ein Medianwert sind, wenn sie in allen Richtungen gemessen werden].

6. Immunochromatographisches Verfahren nach einem der Ansprüche 1 bis 5, wobei (i) zunächst das Trägerpartikel und der Analyt dem Teststück für ein immunochromatographisches Verfahren zugegeben werden und dann das Resonanzpartikel zugegeben wird, (ii) das Resonanzpartikel und das Trägerpartikel mit dem Analyt vermischt werden und die resultierende Mischung dem Teststück für ein immunochromatographisches Verfahren zugegeben wird oder das Resonanzpartikel und das Trägerpartikel in einem Markierungsbereich des Teststücks für ein immunochromatographisches Verfahren enthalten sind.

7. Immunochromatographisches Verfahren nach einem der Ansprüche 1 bis 6, wobei das Trägerpartikel gefärbt oder mit einem Farbstoff markiert ist, oder eine Farbe des Farbstoffs des Trägerpartikels eine Farbe ist, die einer Farbe (Nicht-Absorptionspeak) des Resonanzpartikels ähnelt.

8. Immunochromatographisches Verfahren nach einem der Ansprüche 1 bis 7, wobei das Trägerpartikel einen Fluoreszenzfarbstoff in dem Partikel enthält oder ein Fluoreszenzfarbstoff an eine Oberfläche des Trägerpartikels gebunden ist.

9. Immunochromatographisches Verfahren nach einem der Ansprüche 1 bis 8, wobei der Analyt ein Antigen oder ein Antikörper ist, der in einem lebenden Körper vorhanden ist, oder der Analyt ein pathogener Mikroorganismus und ein Teil davon ist.

10. Immunochromatographisches Testgerät, das in einem Markierungsbereich die Resonanz- und Trägerpartikel gemäß Anspruch 1 umfasst, wobei das Gerät dazu ausgebildet ist, die folgenden Schritte durchzuführen, wenn die Probe auf das Gerät fließt:

den Komplex bildenden Schritt gemäß Anspruch 1,
den Komplex einfangenden Schritt gemäß Anspruch 1, und
die Bereitstellung der nachweisbaren Signale gemäß Anspruch 1.

11. Immunochromatographisches Testgerät nach Anspruch 10, wobei das Resonanzpartikel ein kolloidales Goldpartikel ist oder das Trägerpartikel ein Latexpartikel ist.

12. Immunochromatographisches Testgerät nach Anspruch 10 oder 11, wobei das Teststück ein Trägerpartikel verwendet, an dessen Oberfläche das Resonanzpartikel im Voraus gebunden ist, oder das Teststück ein Trägerpartikel verwendet, in dem das Resonanzpartikel im Voraus eingekapselt ist.

13. Immunochromatographisches Testgerät nach einem der Ansprüche 11 bis 12, wobei die an das Resonanzpartikel gebundene Affinitätssubstanz und die an das Trägerpartikel gebundene Affinitätssubstanz sich an unterschiedliche Bereiche des Analyten binden.

14. Immunochromatographisches Testgerät nach einem der Ansprüche 11 bis 13, wobei die Anzahl der Resonanzpartikel das 12-fache oder mehr der Anzahl der Trägerpartikel beträgt oder die Anzahl der Resonanzpartikel das X-fache oder mehr der Anzahl der Trägerpartikel beträgt [wobei X durch $(3(R + r)^2)/r^2$ mit einem Trägerpartikeldurchmesser = R und einem Resonanzpartikeldurchmesser = r dargestellt wird, mit der Maßgabe, dass die Partikeldurchmesser jeweils ein Medianwert sind, wenn sie in allen Richtungen gemessen werden].

**Revendications**

1. Procédé immunochromatographique utilisant une particule résonante qui provoque une résonance plasmonique de surface et une particule de support qui ne provoque aucune résonance plasmonique de surface et qui est d'une plus grande taille que la particule résonante, sur chacune de laquelle est immobilisée une substance d'affinité qui se lie à un analyte dans un échantillon de prélèvement, le procédé comprenant la formation d'un complexe représenté par particule de support liée à une substance d'affinité qui se lie à l'analyte : analyte : particule résonante liée à une substance d'affinité qui se lie à l'analyte (où « : » indique la liaison) pour ainsi accumuler la particule résonante sur la particule de support, la capture du complexe au niveau d'une région de détection sur une pièce de test pour un procédé immunochromatographique sur laquelle la substance d'affinité qui se lie à un analyte est immobilisée en tant que substance de capture, et la détection de signaux provenant de la particule résonante en raison de la résonance plasmonique de surface et d'un signal provenant de la particule de support pour ainsi détecter l'analyte.

2. Procédé immunochromatographique selon la revendication 1, dans lequel la particule résonante est une particule colloïdale d'or, ou la particule de support est une particule de latex.

3. Procédé immunochromatographique selon l'une quelconque des revendications 1 ou 2, dans lequel le procédé utilise une particule de support ayant la particule résonante liée au préalable à une surface de celle-ci, ou une particule de support ayant la particule résonante encapsulée au préalable dans celle-ci.

4. Procédé immunochromatographique selon l'une quelconque des revendications 1 à 3, dans lequel la substance d'affinité liée à la particule résonante et la substance d'affinité liée à la particule de support se lient à des régions différentes de l'analyte.

5. Procédé immunochromatographique selon l'une quelconque des revendications 1 à 4, dans lequel le nombre de particules résonantes est 12 fois ou plus le nombre de particules de support, ou le nombre de particules résonantes est X fois ou plus le nombre de particules de support [où X est représenté par $(3(R + r)2)/r2$ avec un diamètre de particule de support = R et un diamètre de particule résonante = r, à condition que les diamètres de particules soient chacun une valeur médiane lorsqu'ils sont mesurés dans toutes les directions].

6. Procédé immunochromatographique selon l'une quelconque des revendications 1 à 5, dans lequel (i) la particule de support et l'analyte sont d'abord ajoutés à la pièce de test pour un procédé immunochromatographique, puis la particule résonante est ajoutée, (ii) la particule résonante et la particule de support sont mélangées à l'analyte et le mélange résultant est ajouté à la pièce de test pour un procédé immunochromatographique, ou la particule résonante et la particule de support sont contenues dans une région de marquage de la pièce de test pour un procédé immunochromatographique.

7. Procédé immunochromatographique selon l'une quelconque des revendications 1 à 6, dans lequel la particule de support est colorée ou marquée avec un colorant, ou une couleur du colorant de la particule de support est une couleur similaire à une couleur (pic de non absorption) de la particule résonante.

8. Procédé immunochromatographique selon l'une quelconque des revendications 1 à 7, dans lequel la particule de support comprend un colorant fluorescent dans la particule, ou un colorant fluorescent est lié à une surface de la particule de support.

9. Procédé immunochromatographique selon l'une quelconque des revendications 1 à 8, dans lequel l'analyte est un antigène ou un anticorps présent dans un corps vivant, ou l'analyte est un micro-organisme pathogène et une partie de celui-ci.

10. Dispositif de test immunochromatographique comprenant dans une région de marquage les particules résonante et porteuse telles que spécifiées dans la revendication 1, dans lequel le dispositif est configuré pour effectuer les étapes suivantes lors de l'écoulement de l'échantillon sur ledit dispositif :

l'étape de formation de complexe telle que spécifiée dans la revendication 1,
l'étape de capture de complexe telle que spécifiée dans la revendication 1, et
la fourniture des signaux détectables telle que spécifiée dans la revendication 1.

11. Dispositif de test immunochromatographique selon la revendication 10, dans lequel la particule résonante est une particule colloïdale d'or, ou la particule de support est une particule de latex.

12. Dispositif de test immunochromatographique selon la revendication 10 ou 11, dans lequel la pièce de test utilise une particule de support ayant la particule résonante liée au préalable à une surface de celle-ci, ou la pièce de test utilise une particule de support ayant la particule résonante encapsulée au préalable dans celle-ci.

13. Dispositif de test immunochromatographique selon l'une quelconque des revendications 11 à 12, dans lequel la substance d'affinité liée à la particule résonante et la substance d'affinité liée à la particule de support se lient à des régions différentes de l'analyte.

14. Dispositif de test immunochromatographique selon l'une quelconque des revendications 11 à 13, dans lequel le nombre de particules résonantes est 12 fois ou plus le nombre de particules de support, ou le nombre de particules résonantes est X fois ou plus le nombre de particules de support [où X est représenté par $(3(R + r)2)/r2$ avec un diamètre de particule de support = R et un diamètre de particule résonante = r, à condition que les diamètres de particules soient chacun une valeur médiane lorsqu'ils sont mesurés dans toutes les directions].

# Fig. 1

A

Second liquid

Carrier particle

Capturing antibody
at detection region

Analyte

B

Resonant particle

Carrier particle

Capturing antibody
at detection region

Analyte

# Fig. 2

# Fig. 3

# Fig. 4

| | | | | | |
|---|---|---|---|---|---|
| Conditions | Latex colloid | - | + | + | + |
| | Virus | + | - | + | + |
| | Gold colloid | + | + | - | + |
| Result | Line (color) | - | + (Blue) | + (Blue) | + (Purple) |

# Fig. 5

| | | | | | |
|---|---|---|---|---|---|
| Conditions | Latex colloid | - | + | + | + |
| | Virus | + | - | + | + |
| | Gold colloid | + | + | - | + |
| Result | Line | - | + | + | ++ |

## REFERENCES CITED IN THE DESCRIPTION

### Patent documents cited in the description

- JP 2008286590 A
- WO 2011062157 A
- JP 2017040631 A
- JP 2005233744 A
- JP 2009281760 A
- JP 2018036176 A
- US 2018209965 A1

### Non-patent literature cited in the description

- Development and evaluation of realistic microbioassays in freely suspended droplets on a chip. **RASTOGI VINAYAK et al.** BIOMICROFLUIDICS. AIP, 01 March 2007, vol. 1, 14107-1
- AuNPs amplified magnetophoretic effect for colorimetric detection of nucleic acid. **SHAN CHEN et al.** 10TH IEEE INTERNATIONAL CONFERENCE ON NANO/MICRO ENGINEERED AND MOLECULAR SYSTEMS. IEEE, 07 April 2015, 357-360
- Signal amplification on planar and gel-type sensor surfaces in surface plasmon resonance-based detection of prostate-specific antigen. **BESSELINK G A J.** ANALYTICAL BIOCHEMISTRY. ACADEMIC PRESS, 01 October 2004, vol. 333, 165-173
- **RUILI PAN et al.** Gold nanoparticle-based enhanced lateral flow immunoassay for detection of Cronobacter sakazakii in powdered infant formula. *JOURNAL OF DAIRY SCIENCE,* 01 May 2018, vol. 101 (5), ISSN 0022-0302, 3835-3843
- Gold decorated polystyrene particles for lateral flow immunodetection of Escherichia coli0157:H7. **JIN SEON-AH et al.** MICROCHIMICA ACTA. SPRINGER VIENNA, 05 October 2017, vol. 184, 4879-4886